# DEMANDE DE BREVET EUROPEEN

(11) **EP 0 577 448 A1**
(43) Date de publication de la demande: **05.01.1994**
(21) Numéro de dépôt: 93401425.9
(22) Date de dépôt: 03.06.1993
(51) Int. Cl.: A61M 5/20

(54) **Dispositif d'actionnement automatique d'une seringue**

(30) Priorité: 04.06.1992 FR 9206786
(71) Demandeur: SOCIETE D'ETUDES ET APPLICATIONS TECHNIQUES ( S.E.D.A.T.), F-69450 Irigny (FR)
(72) Inventeur: Arnissolle, Yves, F-69230 St Genis-Laval (FR)
(74) Mandataire: Polus, Camille

(57) **Abrégé**

Ce dispositif (10) comprend un boîtier (12) dans lequel est logée la seringue (14) et un poussoir (46) sollicité élastiquement, destiné à coopérer avec le piston (18) de la seringue (14). Le dispositif comporte de plus des moyens (64,66) de retenue du poussoir (46) en position armée munis de moyens de commande (94) externes au boîtier (12), des moyens (70,72) de libération du poussoir (46), des moyens (22,108) de maintien de la seringue dans une position d'attente dans laquelle l'aiguille (28) de la seringue est escamotée dans le dispositif (10), et des moyens (24,100) de positionnement de la seringue dans une position d'injection dans laquelle l'aiguille (28) fait saillie hors du dispositif (10).

## Description

La présente invention concerne un dispositif d'actionnement automatique d'une seringue.

Pour réaliser l'injection intramusculaire d'un liquide médicamenteux au moyen d'une seringue, le manipulateur procède habituellement en plantant la seringue dans l'organisme et en actionnant le poussoir de la seringue pour injecter le liquide.

Dans certains cas, le patient doit réaliser l'injection lui-même. Lorsque le patient n'est pas expérimenté ou bien lorsque l'endroit où doit être faite l'injection est d'un accès peu commode, l'injection risque d'être mal faite.

L'invention a pour but de réaliser des injections au moyen d'une seringue dans de bonnes conditions, sans que le manipulateur ait lui-même à enfoncer l'aiguille et à actionner le piston de la seringue.

A cet effet, l'invention a pour objet un dispositif d'actionnement automatique d'une seringue, caractérisé en ce qu'il comprend un boîtier de logement de la seringue, un poussoir disposé à l'intérieur du boîtier et sollicité élastiquement dans la direction axiale de la seringue, destiné à coopérer avec le piston de cette seringue, des moyens de retenue du poussoir en position armée, des moyens de libération du poussoir munis de moyens de commande externes au boîtier, des moyens de maintien de la seringue dans une position d'attente dans laquelle l'aiguille de la seringue est escamotée dans le dispositif, et des moyens de positionnement de la seringue dans une position d'injection dans laquelle l'aiguille fait saillie hors du dispositif.

Suivant d'autres caractéristiques de l'invention :
- les moyens de commande des moyens de libération du poussoir comprennent un organe de commande axialement mobile qui fait saillie à travers une ouverture de passage de l'aiguille de la seringue, ménagée à l'extrémité distale du boîtier, cet organe dépassant au delà de l'aiguille en position d'attente de la seringue:
- les moyens de retenue du poussoir comportent au moins deux butées de formes complémentaires, coopérant entre elles, l'une étant solidaire du boîtier et l'autre étant portée par une languette d'armement axiale solidaire du poussoir, cette languette étant souple et déplaçable radialement par déformation élastique, et les moyens de libération du poussoir comprennent au moins deux cames de formes complémentaires, l'une étant reliée aux moyens de commande et l'autre étant portée par la languette d'armement, ces cames coopérant entre elles de façon à déplacer la languette d'armement radialement;
- le dispositif comporte une gaine tubulaire, coulissant axialement dans le boîtier entre deux positions limites, dans laquelle est logée la seringue, l'extrémité distale de la gaine faisant saillie à travers l'ouverture de passage de l'aiguille et formant ledit organe de commande des moyens de libération du poussoir, l'extrémité proximale de la gaine portant la came de libération du poussoir;
- les moyens de maintien de la seringue en position d'attente comprennent un manchon coulissant disposé autour de la seringue, entre cette dernière et la gaine, le manchon étant sollicité élastiquement jusqu'à une position d'attente vers l'extrémité proximale du boîtier par un ressort de raideur telle que la force nécessaire pour s'opposer à la force élastique du ressort soit inférieure à la force nécessaire pour enfoncer le piston de la seringue, ce manchon ayant une extrémité au contact d'une butée solidaire de la seringue;
- les moyens de limitation des déplacements axiaux dans les deux sens opposés de la gaine comprennent des moyens de butée complémentaires ménagés sur la surface externe de la gaine et sur la surface interne du boîtier, et les moyens de positionnement de la seringue en position d'injection comprennent des moyens de butée complémentaires ménagés sur la surface externe du corps de la seringue et sur la surface interne de la gaine;
- le dispositif étant destiné à l'actionnement d'une seringue à corps cylindrique lisse, ladite extrémité du manchon est son extrémité distale et coopère avec l'extrémité distale du corps de la seringue;
- les moyens de limitation des déplacements axiaux dans les deux sens opposés de la gaine comprennent des moyens de butée complémentaires ménagés sur la surface externe de la gaine et sur la surface interne du boîtier, et les moyens de positionnement de la seringue en position d'injection comprennent des moyens de butée complémentaires ménagés sur la surface externe de l'extrémité distale du manchon et sur la surface interne de la gaine;
- le poussoir comporte des moyens de limitation de sa course de détente comprenant au moins une saillie radiale solidaire du poussoir, coulissant dans une rainure axiale ménagée dans la surface interne du boîtier, coopérant par butée avec le bord d'extrémité distale de la rainure lorsque le poussoir est en position de fin de course;
- la saillie radiale est portée par une languette axiale solidaire du poussoir, la languette étant souple et déplaçable radialement par déformation élastique pour escamoter la saillie;
- la languette d'armement et la languette d'escamotage sont alignées axialement en ayant leurs extrémités libres opposées, le bord d'extrémité distale de la rainure coopérant avec la butée de la languette d'armement lorsque le poussoir est en position armée;
- le boîtier comporte un corps et un chapeau amovible fixé au corps par des moyens de verrouillage, le poussoir étant relié au chapeau, le chapeau comportant les moyens de retenue du poussoir en position armée.

Un exemple de réalisation de l'invention sera décrit ci-dessous en se référant aux dessins annexés dans lesquels :
- la figure 1 est une vue en coupe longitudinale d'un dispositif selon l'invention, avec la seringue dans un état d'attente;
- la figure 2 est une vue analogue à la figure 1 montrant le dispositif en service;
- la figure 3 est une vue partielle du dispositif de la figure 1 suivant la flèche 3 de la figure 1;
- la figure 4 est une demi-vue en coupe suivant la ligne 4-4 de la figure 3;
- la figure 5 est une vue à grande échelle en coupe longitudinale d'un poussoir selon un premier mode de réalisation, entrant dans la constitution du dispositif de la figure 1;
- la figure 6 est une vue partielle du poussoir de la figure 5 suivant la flèche 6 de la figure 5;
- la figure 7 est une vue en coupe longitudinale d'une gaine tubulaire entrant dans la constitution du dispositif de la figure 1;
- la figure 8 est une vue à grande échelle en coupe longitudinale d'un manchon entrant dans la constitution du dispositif de la figure 1;
- la figure 9 représente en coupe longitudinale une variante du dispositif selon l'invention, en état d'attente;
- la figure 10 représente la même variante après réalisation de l'injection.

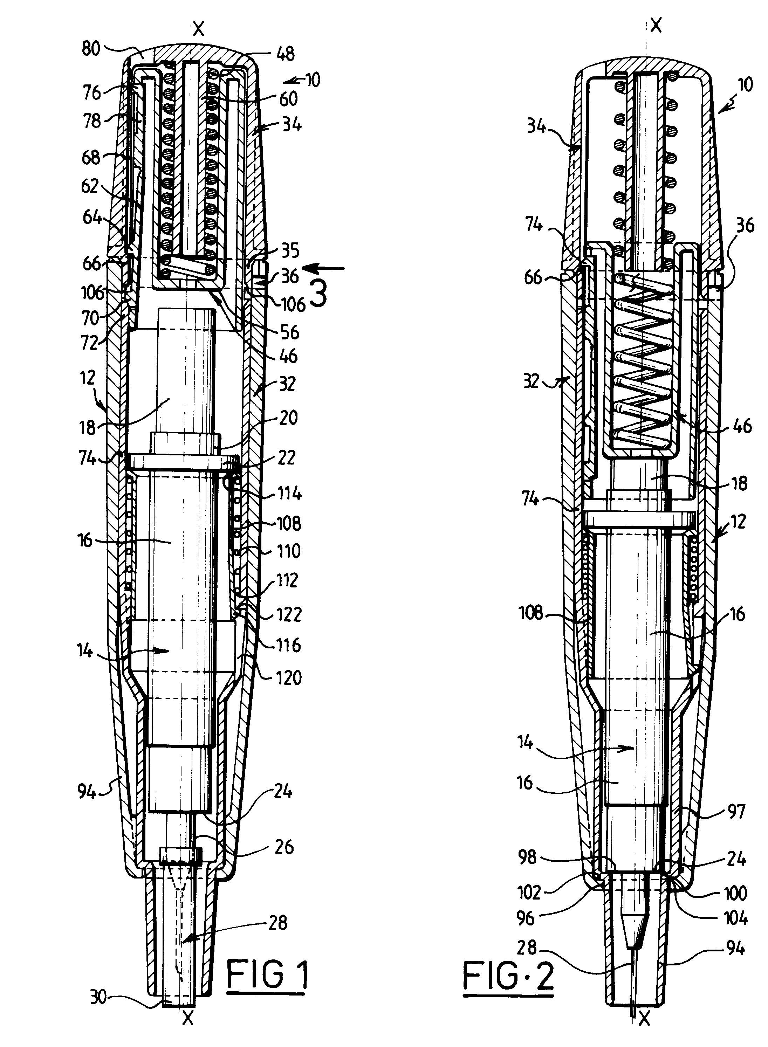

On voit aux figures 1 et 2 un dispositif d'actionnement automatique d'une seringue désigné par la référence générale 10.

Le dispositif 10 comporte un boîtier 12 de forme générale allongée et d'axe longitudinal X-X dans lequel est logée une seringue 14 destinée à l'injection dans un organisme d'un produit médicamenteux.

La seringue 14 comporte un corps 16 dans lequel coulisse un piston 18. L'extrémité proximale 20 du corps 16 comporte un collet 22. L'extrémité distale 24 du corps 16 est prolongée par un porte-aiguille 26 muni à son extrémité libre d'une aiguille 28.

Sur la figure 1 on a représenté un capuchon 30 recouvrant l'aiguille 28, emmanché par son extrémité ouverte sur le porte-aiguille 26.

Le capuchon 30 fait saillie à l'extérieur du boîtier 12, ce qui permet de l'enlever préalablement à la mise en service du dispositif 10.

Sur la figure 2 le capuchon 30 a été enlevé.

Le boîtier 12 comprend un corps de boîtier 32 et un chapeau 34, solidaire du corps 32 de boîtier, formant l'extrémité proximale du boîtier 12.

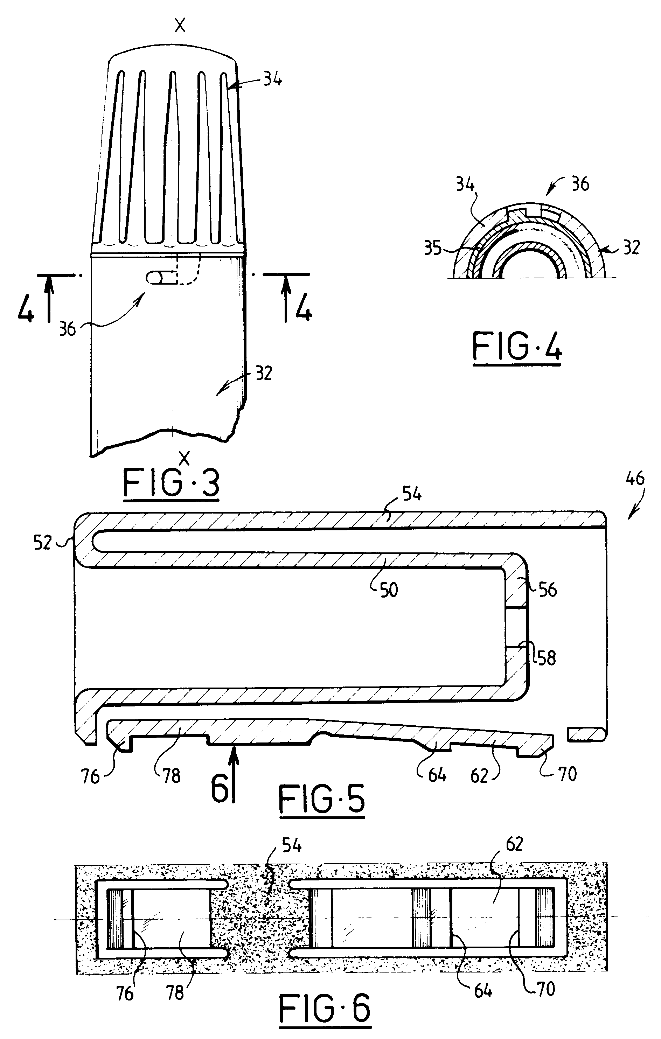

L'extrémité ouverte du chapeau 34 forme un embout cylindrique 35 emmanché dans l'extrémité proximale du corps 32 de boîtier en étant fixée sur celui-ci de façon amovible par des doubles moyens de verrouillage 36 du type baïonnette, diamétralement opposés, qui sont montrés plus en détail aux figures 3 et 4.

Le dispositif d'actionnement 10 comporte un poussoir 46 destiné à déplacer la seringue 14 suivant son axe X-X et à actionner le piston 18 de la seringue.

La seringue 14 est déplaçable axialement dans le boîtier 12 entre une position d'attente dans laquelle l'aiguille 28 est escamotée à l'intérieur du boîtier 12, comme cela est représenté à la figure 1, et une position d'injection dans laquelle l'aiguille 28 fait saillie à l'extérieur du boîtier 12, comme cela est représenté à la figure 2.

Le poussoir 46 est montré plus en détail aux figures 5 et 6. Ce poussoir 46 a une forme générale cylindrique et est sollicité élastiquement dans la direction axiale X-X du boîtier 12 par un ressort 48.

Le poussoir 46 comporte un corps 50 central, de forme générale tubulaire, relié par son extrémité proximale 52 à une jupe périphérique 54 entourant le corps 50 de poussoir. La jupe 54 est guidée axialement en étant au contact des parois internes du chapeau 34 et du corps 32 de boîtier.

L'extrémité distale du corps 50 de poussoir est délimitée par une paroi 56, perpendiculaire à l'axe X-X, munie d'un orifice axial 58 et destinée à coopérer avec le piston 18 de la seringue.

Le ressort 48 est disposé à l'intérieur du corps 50 de poussoir entre le fond du chapeau 34 et la paroi 56. Le ressort 48 est guidé par un tube 60 solidaire par une de ses extrémités du fond du chapeau 34 et s'étendant axialement à l'intérieur des spires du ressort 48.

La jupe 54 du poussoir 46 comporte des moyens d'armement et de libération du poussoir 46 et des moyens de positionnement de fin de course du poussoir 46.

Les moyens d'armement et de libération du poussoir sont portés par trois languettes 62 souples, dont une seule est représentée sur les figures, s'étendant axialement et réparties sur le pourtour de la jupe 54 en étant espacées entre elles de 120°.

Les languettes 62 sont venues de matière avec la jupe 54 en étant solidaires de celle-ci par une de leurs extrémités et sont déplaçables radialement par déformation élastique. Chaque languette 62 comporte une saillie radiale 64 formant butée de retenue du poussoir 46 en position armée. La butée 64 de retenue est disposée entre l'extrémité fixe et l'extrémité libre de la languette 62.

La butée 64 de retenue coopère, en position d'armement du poussoir 46, avec une butée 66 de forme complémentaire ménagée sur la surface interne du chapeau 34, comme cela est représenté à la figure 1.

La butée 66 du chapeau 34 est délimitée par le bord d'extrémité distale d'une rainure axiale 68 ménagée dans la surface interne du chapeau 34.

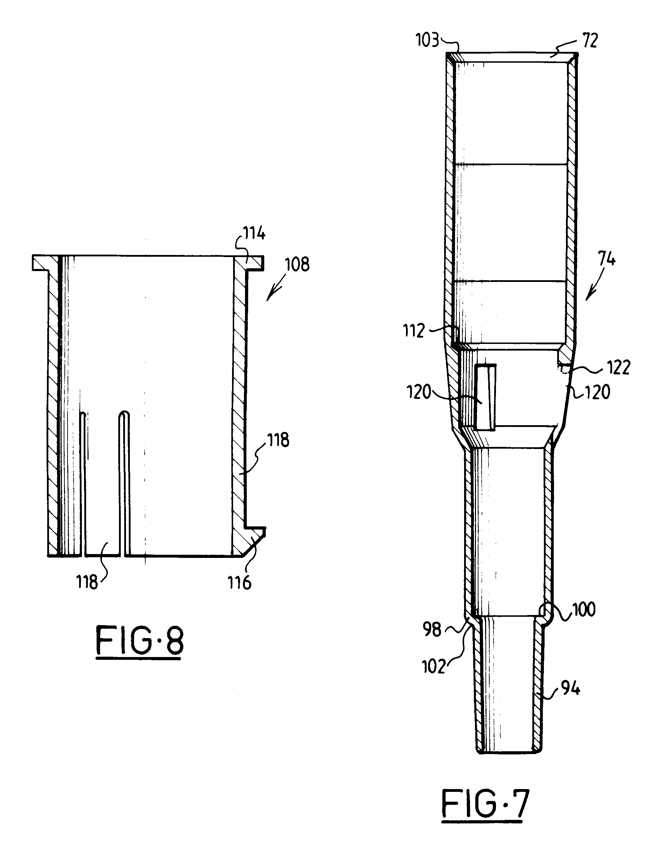

L'extrémité libre des languettes 62 comporte une came 70 de libération du poussoir 46. Cette came 70 coopère avec une came 72 portée par un organe de commande 74 qui est représenté plus en détail à la figure 7 et qui sera décrit ultérieurement.

Les moyens de positionnement de fin de course du poussoir 46 comportent trois saillies 76 radiales formant butée, dont une seule est représentée sur les figures. Les butées 76 sont réparties sur le pourtour de la jupe 54 du poussoir 46 en étant espacées entre elles de 120°.

Chaque butée 76 de fin de course est portée par une languette 78 souple, s'étendant axialement et solidaire de la jupe 54 du poussoir 46.

La languette 78 est venue de matière avec la jupe 54 en étant solidaire de celle-ci par une de ses extrémités. La languette 78 est déplaçable radialement par déformation élastique de façon à escamoter la butée 76 notamment au moment du montage du dispositif 10.

Chaque languette 78 d'escamotage est alignée avec une languette 62 d'armement, les extrémités libres de la languette 78 d'escamotage et de la languette 62 d'armement étant opposées.

La butée 76 de fin de course coulisse dans la rainure 68 et coopère, en position de fin de course du poussoir 46, avec le bord 66 d'extrémité distale de la rainure 68, comme cela est montré à la figure 2.

L'extrémité proximale de la rainure 68 est délimitée par une ouverture 80 traversant le fond du chapeau 34 et permettant notamment de contrôler visuellement la position du poussoir 46 avant et après son utilisation.

On décrira maintenant l'organe 74 de commande de la libération du poussoir 46 en regard des figures 1,2 et 7.

L'organe 74 de commande forme une gaine déplaçable axialement dans le boîtier 12 dans laquelle est logée la seringue 14.

La gaine 74 est constituée de tronçons successifs de diamètres décroissants vers son extrémité distale. Un tronçon 94 délimitant l'extrémité distale de la gaine 74 fait saillie à l'extérieur du boîtier 12 à travers une ouverture 96 de passage de l'aiguille 28 ménagée à l'extrémité distale du boîtier 12.

Le tronçon 94 est relié par un col 98 au reste de la gaine 74.

Le col 98 délimite un épaulement interne 100 et un épaulement externe 102. L'épaulement interne 100 coopère avec l'extrémité distale du corps 16 de la seringue 14 lorsque celle-ci est en position d'injection, comme représentée à la figure 2. L'extrémité proximale 103 de la gaine 74 comporte un chanfrein formant la came 72 de commande de la libération du poussoir 46.

Le déplacement axial de la gaine 74 vers l'extrémité distale du boîtier 12 est limité par des butées complémentaires formées, l'une par l'épaulement externe 102 du col 98 et l'autre par un épaulement interne 104 délimité par la paroi du boîtier autour de l'ouverture 96.

Le déplacement axial de la gaine 74 vers l'extrémité proximale du boîtier 12 est limité par des butées complémentaires formées, l'une par l'extrémité libre 106 de l'embout 42 du chapeau 34, et l'autre par l'extrémité proximale 103 de la gaine 74.

La seringue 14 est maintenue en position d'attente à l'intérieur du boîtier 12 par un manchon 108 montré plus en détail à la figure 8. Le manchon 108 est disposé autour du corps 16 de la seringue 14, entre celle-ci et la gaine 74.

Un ressort 110, disposé autour du manchon 108 entre celui-ci et la gaine 74, sollicite élastiquement le manchon 108 vers l'extrémité proximale du boîtier 12. Le ressort 110 est disposé entre un épaulement 112 de la surface interne de la gaine 74 et une collerette extérieure 114 délimitant l'extrémité proximale du manchon 108.

Le ressort 110 a une raideur faible de manière que la force nécessaire pour s'opposer à la force élastique du ressort soit inférieure à la force nécessaire pour enfoncer le piston 18 de la seringue. La raideur du ressort 110 est donc inférieure à celle du ressort 48 sollicitant le poussoir 46.

L'extrémité distale du manchon 108 comporte trois saillies 116 radiales, dont une seule est représentée sur les figures. Chaque saillie 116 radiale est portée par l'extrémité libre d'une languette 118 souple s'étendant axialement et venue de matière avec le manchon 108. Les languettes 118 sont réparties sur le pourtour du manchon 108 en étant espacées entre elles de 120°. Les languettes 118 sont déplaçables radialement par déformation élastique et permettent l'escamotage des saillies 116, en particulier au moment du montage du manchon 108 dans le dispositif 10.

Les saillies 116 radiales coulissent dans des lumières 120 axiales ménagées dans la paroi de la gaine 74.

Les saillies 116 limitent la course du manchon 108 en coopérant par butée avec les bords 122 d'extrémité proximale des lumières 120. La collerette 114 du manchon 108 coopère par butée avec le collet 22 du corps 16 de la seringue de façon à maintenir cette dernière dans sa position d'attente, comme cela est montré à la figure 1.

On décrira maintenant le fonctionnement du dispositif 10 d'actionnement automatique de la seringue.

La seringue 14 est introduite dans le boîtier 12 après avoir séparé le chapeau 34 du corps 32 de boîtier. On arme ensuite le poussoir 46 en l'enfonçant vers le fond du chapeau 34 et en s'opposant à la force élastique du ressort 48, de façon à faire buter les saillies 64 des languettes 62 d'armement contre les bords 66 d'extrémité distale des rainures 68.

Une fois le poussoir 46 armé dans le chapeau 34, on fixe ce chapeau 34 sur le corps 32 de boîtier grâce aux moyens de verrouillage 36.

La seringue 14 est maintenue en position d'attente par le manchon 108 qui est sollicité élastiquement au maximum de sa course par le ressort 110.

Le dispositif 10 est alors dans son état d'attente tel que représenté à la figure 1.

Pour réaliser l'injection, on enlève le capuchon 30 recouvrant l'aiguille 28. Tant qu'on n'a pas commandé la libération du poussoir 46, l'aiguille 28 est escamotée dans la gaine 74, comme cela est représenté à la figure 1.

Lorsque l'on veut réaliser l'injection, on applique l'extrémité distale de la gaine 74 contre la partie de l'organisme où l'on veut planter l'aiguille 28. En appuyant légèrement sur le boîtier 12, en direction de son extrémité distale, la gaine 74 se déplace relativement au boîtier 12, ce qui a pour effet de faire coopérer entre elles la came 72 de l'extrémité proximale de la gaine 74 et les cames 70 des languettes 62 d'armement. Les languettes 62 sont déplacées radialement vers l'intérieur du boîtier et les butées 64 de retenue des languettes 62 sont libérées.

L'ensemble du poussoir 46 se déplace alors rapidement vers l'extrémité distale du boîtier et heurte le piston 18 de la seringue.

Etant donné que la raideur du ressort 110 du manchon 108 est faible, l'ensemble de la seringue 14 est déplacé rapidement vers l'extrémité distale du boîtier 12, sans que le piston 18 ne s'enfonce dans le corps 16 de la seringue. Par suite, l'aiguille se plante dans le patient.

Lorsque l'extrémité distale 24 du corps 16 de la seringue est en butée contre l'épaulement 100 de la gaine 74 et l'épaulement 102 de celle-ci en butée contre l'épaulement 104 du boîtier, le poussoir 46 enfonce le piston 18 dans le corps 16 de la seringue. Le liquide contenu dans la seringue est alors injecté dans l'organisme.

Le piston 18 s'enfonce dans le corps 16 de la seringue jusqu'à ce que les saillies 76 de fin de course du poussoir 46 soient en butée contre les butées 66 des rainures 68 du chapeau, comme cela est représenté à la figure 2.

Lorsque le poussoir 46 est en fin de course, l'injection est terminée et on retire l'aiguille 28 de l'organisme en tirant sur le boîtier 12.

Pour recharger le dispositif 10, on déverrouille le chapeau 34 du corps 32 de boîtier pour remplacer la seringue vide par une seringue pleine.

Le dispositif selon l'invention comporte de nombreux avantages.

Il permet à un patient d'effectuer sur lui-même des injections de produits médicamenteux sans avoir lui-même à planter la seringue et à actionner son piston.

Le poussoir disposé à l'intérieur du boîtier permet de planter la seringue dans l'organisme puis d'injecter le liquide de la seringue progressivement dans cet organisme.

Le poussoir est relié au chapeau du boîtier que ce soit dans sa position armée ou dans sa position détendue. Ceci permet, en retirant le chapeau du boîtier, de dégager immédiatement l'accès au logement de la seringue dans le boîtier.

Le remplacement de la seringue vide dans le dispositif est très simple, ce qui permet une réutilisation très facile du dispositif.

La commande de la libération du poussoir par un organe prolongeant l'ouverture de passage de l'aiguille permet de planter la seringue à l'endroit précis de l'organisme où l'on veut réaliser l'injection.

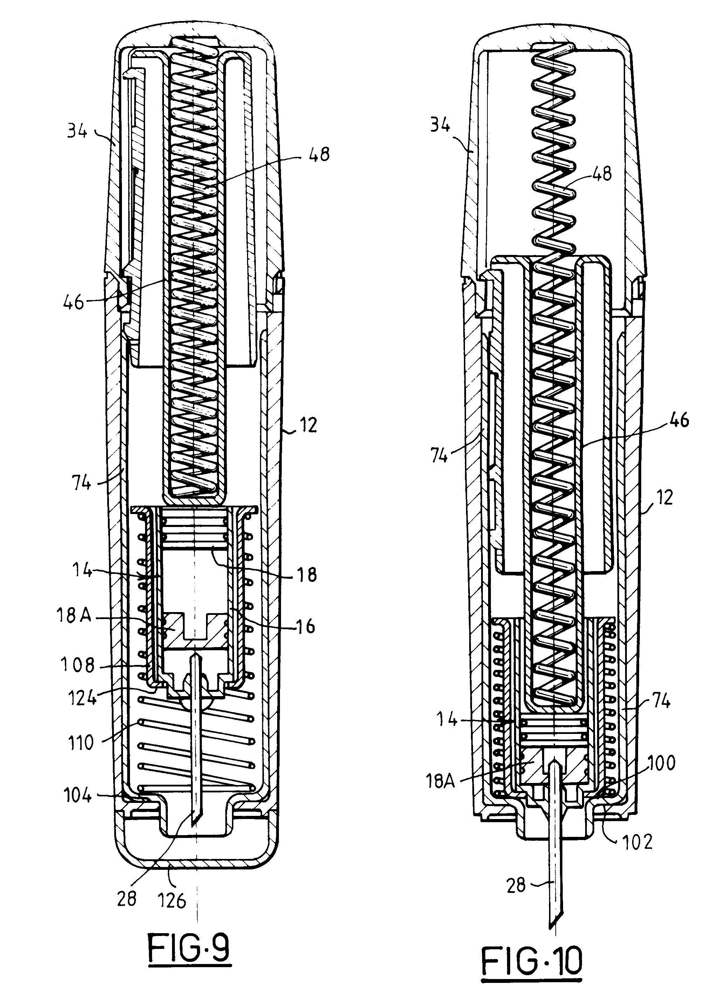

Les figures 9 et 10 montrent un dispositif d'actionnement similaire dans l'ensemble à celui des figures 1 à 8, mais modifié pour servir à l'actionnement automatique d'un autre type de seringue 14 ayant un corps 16 cylindrique lisse, c'est-à-dire dépourvu du collet 22. Dans cet exemple, le corps 16 contient initialement un piston d'actionnement 18 à son extrémité proximale, et un piston auxiliaire perforable 18A à peu près à mi-longueur, ces deux pistons délimitant entre eux une chambre emplie du liquide à injecter. L'extrémité proximale de l'aiguille 28 fait saillie dans le corps 16 et est biseautée.

La variante des figures 9 et 10 diffère de celle des figures 1 à 8 par le fait que le manchon 108 comporte une collerette intérieure 124 à son extrémité distale et entoure entièrement le corps 16, lequel bute sur cette collerette pour son positionnement axial. On a également représenté un couvercle de protection amovible 126, en forme de cuvette, emboîté à l'extrémité distale du boîtier 12.

Lorsque le poussoir 46 est libéré comme précédemment, il vient heurter le piston 18 et déplace vers l'avant l'ensemble seringue 14-manchon 108, en comprimant le ressort 110, jusqu'à ce que la collerette 124 du manchon bute contre l'épaulement 104 du boîtier 12 par l'intermédiaire des épaulements 100 et 102 de la gaine 74.

Puis, le ressort 48 continuant à se détendre, le poussoir 46 pénètre dans le corps 16 de la seringue en déplaçant vers l'avant le piston 18 et, par l'intermédiaire du liquide à injecter, le piston 18A. Ce dernier est alors perforé par l'aiguille 28, et l'injection est réalisée. La position finale est celle représentée sur la figure 10, la figure 9 représentant la position d'attente.

## Revendications

1. Dispositif d'actionnement automatique d'une seringue, caractérisé en ce qu'il comprend un boîtier (12) de logement de la seringue, un poussoir (46) disposé à l'intérieur du boîtier (12) et sollicité élastiquement dans la direction axiale de la seringue (14), destiné à coopérer avec le piston (18) de cette seringue (14), des moyens (64,66) de retenue du poussoir (46) en position armée, des moyens (70,72) de libération du poussoir (46) munis de moyens (94) de commande externes au boîtier (12), des moyens (22,108) de maintien de la seringue (14) dans une position d'attente dans laquelle l'aiguille (28) de la seringue est escamotée dans le dispositif, et des moyens (24,100) de positionnement de la seringue dans une position d'injection dans laquelle l'aiguille (28) fait saillie hors du dispositif.

2. Dispositif selon la revendication 1, caractérisé en ce que les moyens de commande des moyens (70,72) de libération du poussoir (46) comprennent un organe de commande axialement mobile (94) qui fait saillie à travers une ouverture (96) de passage de l'aiguille (28) de la seringue (14), ménagée à l'extrémité distale du boîtier (12), cet organe dépassant au delà de l'aiguille (28) en position d'attente de la seringue.

3. Dispositif selon la revendication 1 ou 2, caractérisé en ce que les moyens de retenue du poussoir comportent au moins deux butées (64,66) de formes complémentaires, coopérant entre elles, l'une étant solidaire du boîtier (12) et l'autre étant portée par une languette (62) d'armement axiale solidaire du poussoir (46), cette languette (62) étant souple et déplaçable radialement par déformation élastique, et en ce que les moyens de libération du poussoir comprennent au moins deux cames (70,72) de formes complémentaires, l'une étant reliée aux moyens (94) de commande et l'autre étant portée par la languette (62) d'armement, ces cames (70,72) coopérant entre elles de façon à déplacer la languette (62) d'armement radialement.

4. Dispositif selon les revendications 2 et 3 prises ensemble, caractérisé en ce qu'il comporte une gaine tubulaire (74), coulissant axialement dans le boîtier (12) entre deux positions limites, dans laquelle est logée la seringue (14), l'extrémité distale (94) de la gaine (74) faisant saillie à travers l'ouverture (96) de passage de l'aiguille et formant ledit organe de commande des moyens (70,72) de libération du poussoir (46), l'extrémité proximale (103) de la gaine (74) portant la came (72) de libération du poussoir (46).

5. Dispositif selon la revendication 4, caractérisé en ce que les moyens de maintien de la seringue (14) en position d'attente comprennent un manchon (108) coulissant disposé autour de la seringue (14), entre cette dernière et la gaine (74), le manchon (108) étant sollicité élastiquement jusqu'à une position d'attente vers l'extrémité proximale du boîtier (12) par un ressort (110) de raideur telle que la force nécessaire pour s'opposer à la force élastique du ressort (110) soit inférieure à la force nécessaire pour enfoncer le piston (18) de la seringue (14), ce manchon ayant une extrémité (114) au contact d'une butée (22) solidaire de la seringue (14).

6. Dispositif selon la revendication 4 ou 5, caractérisé en ce que les moyens (102,103,104,106) de limitation des déplacements axiaux dans les deux sens opposés de la gaine (74) comprennent des moyens (102, 103,104,106) de butée complémentaires ménagés sur la surface externe de la gaine (74) et sur la surface interne du boîtier (12), et en ce que les moyens de positionnement de la seringue en position d'injection comprennent des moyens (24,100) de butée complémentaires ménagés sur la surface externe du corps (16) de la seringue et sur la surface interne de la gaine (74).

7. Dispositif selon la revendication 5, pour l'actionnement d'une seringue à corps cylindrique lisse, caractérisé en ce que ladite extrémité du manchon (108) est son extrémité distale et coopère avec l'extrémité distale du corps (16) de la seringue (14).

8. Dispositif selon la revendication 7, caractérisé en ce que les moyens (102,103,104,106) de limitation des déplacements axiaux dans les deux sens opposés de la gaine (74) comprennent des moyens (102, 103,104,106) de butée complémentaires ménagés sur la surface externe de la gaine (74) et sur la surface interne du boîtier (12), et en ce que les moyens de positionnement de la seringue en position d'injection comprennent des moyens (124, 100) de butée complémentaires ménagés sur la surface externe de l'extrémité distale du manchon (108) et sur la surface interne de la gaine (74).

9. Dispositif selon l'une quelconque des revendications 1 à 8, caractérisé en ce que le poussoir (46) comporte des moyens (76,66) de limitation de sa course de détente comprenant au moins une saillie (76) radiale solidaire du poussoir (46), coulissant dans une rainure (68) axiale ménagée dans la surface interne du boîtier (12), coopérant par butée avec le bord (66) d'extrémité distale de la rainure (68) lorsque le poussoir (46) est en position de fin de course.

10. Dispositif selon la revendication 9, caractérisé en ce que la saillie (76) radiale est portée par une languette (78) axiale solidaire du poussoir (46), la languette (78) étant souple et déplaçable radialement par déformation élastique pour escamoter la saillie (76).

11. Dispositif selon les revendications 3 et 10 prises ensemble, caractérisé en ce que la languette (62) d'armement et la languette (78) d'escamotage sont alignées axialement en ayant leurs extrémités libres opposées, le bord (66) d'extrémité distale de la rainure (68) coopérant avec la butée (64) de la languette (62) d'armement lorsque le poussoir (46) est en position armée.

12. Dispositif selon l'une quelconque des revendications précédentes, caractérisé en ce que le boitier (12) comporte un corps (32) et un chapeau amovible (34) fixé au corps par des moyens (36) de verrouillage, le poussoir (46) étant relié au chapeau (34), le chapeau comportant les moyens (64,66) de retenue du poussoir (46) en position armée.
